(19) **Europäisches Patentamt European Patent Office Office européen des brevets**

(11) **EP 4 609 715 A1**

(12) **EUROPEAN PATENT APPLICATION**

(43) Date of publication:
03.09.2025 Bulletin 2025/36

(21) Application number: 25154974.7

(22) Date of filing: 30.01.2025

(51) International Patent Classification (IPC):
*A01N 65/22* (2009.01)     *A01P 3/00* (2006.01)

(52) Cooperative Patent Classification (CPC):
A01N 65/22; A01P 3/00

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 31.01.2024 PL 44766924

(71) Applicants:
• Uniwersytet Mikolaja Kopernika w Toruniu
87-100 Torun (PL)

• Uniwersytet Marii Curie Sklodowskiej
20-031 Lublin (PL)

(72) Inventors:
• Zaluski, Daniel
Warszawa (PL)
• Ptaszynska, Aneta
Swidnik (PL)
• Gebalski, Jakub
Bydgoszcz (PL)

(54) **ETHANOL EXTRACT FROM FRESH LYOPHILIZED ROOT OF SCUTELLARIA BAICALENSIS TO INCREASE THE IMMUNITY OF BEES AND COMBAT NOSEMOSIS**

(57) The subject of the invention is an ethanol extract from fresh lyophilized root of *Scutellaria baicalensis* for use in preparations increasing the immunity of bees and combating nosemosis, ethanol extract from fresh lyophilized root of *Scutellaria baicalensis* and method of obtaining ethanol extract from fresh lyophilised root of *Scutellaria baicalensis*.

EP 4 609 715 A1

**Description**

The subject of the invention is an ethanol extract from fresh lyophilised root of *Scutellaria baicalensis* for use in preparations increasing the immunity of bees and combatinh nosemosis, ethanol extract of fresh lyophilized root of *Scutellaria baicalensis* and method of obtaining ethanol extract from fresh lyophilized root of *Scutellaria baicalensis.*

From the patent description PL241381is known an extract prepared with an aqueous ethanol solution from the roots, stems or leaves of plants belonging to the genus *Cannabis,* such as *Cannabis sativa, Cannabis indica* and *Cannabis ruderalis,* which immunizes honeybees against the damaging effects of neonicotinoid insecticides, the most commonly used being imidacloprid and acetamiprid, and which has been shown to be active against nosemosis caused by infection with spores of the fungus *Nosema spp.*

From the patent description PL241380 is known an extract prepared with hot water before boiling from roots, stems or leaves of plants belonging to the genus *Cannabis* such as *Cannabis sativa, Cannabis indica* and *Cannabis ruderalis,* protecting honeybees against the damaging effects of neonicotinoid insecticides, the most commonly used being imidacloprid and acetamiprid, and also showing activity against nosema caused by infection with spores of the fungus *Nosema spp.* The patent no. PL231692 describes a formulation for the treatment of microsporidiosis, in particular nosema in bees, caused by infection with fungi such as *Nosema apis* or *Nosema ceranae,* based on commonly used food with the addition of porphyrin compounds obtained by introducing amino acid and peptide groupings, meso-substituted porphyrins or chlorophyll derivatives into the porphyrin structure.

The patent no. PL 232685 describes a formulation in the form of chloroform, ethanol or aqueous extracts made from the roots, stems, leaves or fruits of plants belonging to the genus *Eleutherococcus,* which are used both to treat nosemosis in honey bees caused by infection with fungi such as *Nosema apis* or *Nosema ceranae* and to improve their immunity.

The subject of the invention differs from previous ones in that it is prepared from raw material (root) lyophilized immediately after harvesting. After lyophilization of the raw material, two extraction techniques were used: ultrasound-assisted extraction (UAE) and maceration, using a 75% aqueous ethanol as solvent. This way of processing the plant material allows the concentration of active compounds determining the therapeutic effect of the extract obtained from it to be maintained. It is important to use an aqueous ethanol solution to dissolve and extract the flavonoids and phenolic acids.

Surprisingly, the use of the root of *Scutellaria baicalensis* has been shown to increase bee immunity and offers the possibility of controlling nosema.

The aim of the invention is to develop an ethanol extract such that it can be used in bees to increase their immunity and to control nosema.

The essence of the invention is an ethanol extract from the fresh lyophilized root of *Scutellaria baicalensis* for use in preparations increasing the immunity of bees and combating nosema, which is prepared from the roots of the *Scutellaria baicalensis* plant.

Ethanol extract of fresh lyophilised *Scutellaria baicalensis* root obtained by UAE extraction technique to increase bee immunity and control nosema contains:

- TPC 51.42 mg/g of extract
- TFC 5.92 mg/g of extract
- TPAC 1.09 mg/g of extract,

where TPC stands for total polyphenols, TFC stands for total flavonoids and TPAC stands for total phenolic acids.

Preferably, it also includes:

- baicalein from 0.155 to 0.290 mg/ml
- baicalin from 0.244 to 0.589 mg/ml
- vogoside from 0.234 to 0.761 mg/ml
- vogonoside from 1.523 to 2.601 mg/ml

The essence of the invention is also a method of obtaining an ethanol extract from fresh lyophilized root of *Scutellaria baicalensis* obtained by UAE extraction technique to increase bees immunity and to control nosema by extracting the ground root of *Scutellaria baicalensis* with 75% ethanol at a temperature of 18 to 24°C over a period of 16 to 36 hours, preferably 24 hours, then subjecting to ultrasounds in the range of 30 to 50 kHz, preferably 40 kHz, for 10 to 25 minutes, preferably 15 minutes, at a temperature of 30 to 45°C, preferably 37°C, filtering under reduced pressure in the range of 0.1 to 2 bar and extracted with 75% ethanol, subjecting to ultrasounds in the range of 30 to 50 kHz, preferably 40 kHz, for 10 to 25 minutes, preferably 15 minutes, at a temperature of 30 to 45°C, preferably 37°C, and again subjecting the resulting extract to the entire process, two to ten times, preferably three, and then concentrating under vacuum in an evaporator at a temperature of 20 to 45°C, preferably 30°C, and freeze-drying the resulting concentrated extract over a period of 24 to 240 hours, preferably 72 hours, at a temperature of -30 to -5°C, preferably -15°C.

Preferably, 50 to 150 ml, preferably 100 ml of ethanol is used for every 10 g of ground root.

The essence of the invention is also an ethanol extract from fresh lyophilized root of the *Scutellaria baicalensis* obtained by maceration technique to increase bee immunity and to control nosema, containing:

- TPC 58.78 mg/g of extract
- TFC 6.68 mg/g of extract
- TPAC 1.17 mg/g of extract

where TPC stands for total polyphenols, TFC stands for total flavonoids and TPAC stands for total phenolic acids

Preferably, also includes:

- baicalein from 0.244 to 0.570 mg/ml
- baicalin from 0.3423 to 0.671 mg/ml
- vogoside from 0.487 to 0.946 mg/ml
- vogonoside from 1.756 to 2.683 mg/ml.

The essence of the invention is also a method of obtaining an ethanol extract from fresh lyophilized *Scutellaria baicalensis* root obtained by maceration technique for increasing bee immunity and controling nosema, whereby the ground *Scutellaria baicalensis* root is extracted with 75% ethanol at 18 to 24°C for 16 to 36h, preferably 24h, filtered under reduced pressure in the range of 0.1 to 2 bar and extracted with 75%, then vacuum-concentrated in an evaporator at 20 to 45°C, preferably 30°C, and the extract concentrated in this way is lyophilized for 24 to 240 h, preferably 72h, at -30 to -5°C, preferably -15°C. Preferably 50 to 150 ml, preferably 100 ml of ethanol, is used for each 10 g of ground root.

Example I.

Ethanol extract of fresh lyophilized root of *Scutellaria baicalensis* for use in preparations to increase bee immunity and to control nosema

Diagram 1. Total content of phenolic compounds in lyophilised freshly harvested raw material [mg/g of extract]. TPC — total polyphenols, TFC — total flavonoids, TPAC — total phenolic acids. UAE.L - extract obtained by ultrasound-assisted

ex

extraction. M.L — extract obtained by maceration.

traction. M.L - extract obtained by maceration.

Table 1. Total content of phenolic compounds in lyophilized freshly harvested raw material [mg/g of extract]. TPC - total polyphenols, TFC - total flavonoids, TPAC - total phenolic acids. UAE.L - extract obtained by ultrasound-assisted extraction. M.L - extract obtained by maceration.

|  | TPC |  | TFC |  | TPAC |  |
|---|---|---|---|---|---|---|
|  | MEAN | SD | MEAN | SD | MEAN | SD |
| UAE.L | 51.4277 6 | 3.38614 2 | 5.92439 8 | 0.27190 2 | 1.09268 8 | 0.15367 3 |
| M.L | 58.7826 5 | 3.50311 5 | 6.68073 7 | 0.31526 6 | 1.17502 2 | 0.10875 9 |

The method of obtaining an ethanol extract from the root of the *Scutellaria baicalensis* plant by maceration technique consists of extracting 10 g of ground raw material in the form of fresh lyophilised *Scutellaria baicalensis* root with 100 ml of 75% ethanol at room temperature for 24 hours. After this time, the extract was filtered under the pressure reduced to 2 bar, and the raw material was washed three times with 50 ml of 75% ethanol and vacuum concentrated using a vacuum evaporator at 30°C. In the final step, the concentrated extract was lyophilized for 3 days at a temperature of -15°C. The method resulted in an extract with the values given in Table 1 and in Diagram 1.

The method of obtaining the extract consists of extracting 10 g of ground raw material with 100 ml of 75% ethanol at room temperature over a period of 24 hours. After this time, the mixture was subjected to ultrasound (40 kHz) for 15 minutes, at a temperature of 37°C. Then, the extract was filtered under reduced pressure to 1 bar and the raw material was extracted again with 50 ml of 75% ethanol in the presence of ultrasounds (40 kHz) for a further 15 minutes, at a temp. of 37°C. The procedure was repeated three times. The extract was concentrated using a vacuum evaporator at 30°C. In the final step, the concentrated extract was lyophilized for 3 days at -15°C. The method resulted in an extract with the values given in Table 1 and in Diagram 1.

**Determination of total polyphenols by the Folin-Ciocalteu method**

Extract in the amount of 25 $\mu$l, 25 $\mu$l of triple-diluted FC reagent and 200 $\mu$l of distilled water were added to a 96-well plate. The plate was incubated for 5 minutes. Then 25 $\mu$l of saturated $Na_2CO_3$ solution was added to all wells. The plate prepared in this way was incubated for 60 minutes in the dark, at room temperature. The absorbance of the analysed samples was measured at $\lambda$ = 750 nm. Each assay was performed in triplicate.

**Determination of total flavonoids by the Al(III) ion method**

Extract in the amount of 25 $\mu$l, 75 $\mu$l of ethanol, 10 $\mu$l of aluminium trichloride solution, 10 $\mu$l of potassium acetate solution and 130 $\mu$l of distilled water were added to a 96-well plate. The plate was then incubated for 30 minutes in the dark, at room temperature. The absorbance of the analysed samples was measured at $\lambda$ = 510 nm. Each assay was performed in triplicate. **Determination of total phenolic**

**acids by the Arnov method**

Extract in the amount of 25 $\mu$l, 150 $\mu$l of distilled water, 25 $\mu$l of HCl solution, 25 $\mu$l of Arnov's reagent and 25 $\mu$l of NaOH solution were added to a 96-well plate. The absorbance of the analysed samples was measured at $\lambda$ = 492 nm. Each assay was performed in triplicate.

## IC50 HYAL — lyophilisation

Diagram 2. Inhibition of hyaluronidase expressed as IC50 value [ug/ml] — freshly harvested raw material subjected to lyophilization. UAE.L — an extract obtained by o ultrasound-assisted extraction. M.L — extract obtained by maceration.

Table 2. Inhibition of hyaluronidase expressed as IC50 value [ug/ml] - freshly harvested raw material subjected to lyophilization. UAE.L - an extract obtained by ultrasound-assisted extraction. M.L - extract obtained by maceration.

|       | MEAN   | SD   |
|-------|--------|------|
| UAE.L | 505.12 | 9.23 |
| M.L   | 332.58 | 5.91 |

**Test for inhibition of bovine hyaluronidase activity**

The test was carried out in a 96-well plate, using four concentrations of each extract: 1, 0.5, 0.1 and 0.01 mg/ml. To each well, 10 $\mu$l of extract, 15 $\mu$l of acetate buffer, 25 $\mu$l of incubation buffer and 25 $\mu$l of hyaluronidase solution ($T_S$) were added. First control sample with extract without enzyme ($T_H$): 10 $\mu$l of extract, 40 $\mu$l of acetate buffer and 25 $\mu$l of incubation buffer. Second control sample with enzyme without extract ($T_C$): 10 $\mu$l of 50% DMSO, 15 $\mu$l acetate buffer, 25 $\mu$l incubation buffer and 25 $\mu$l enzyme. All assays were made in triplicate. The plates prepared in this way were incubated for 10 minutes at 37°C. Then, 25 $\mu$l of hyaluronic acid solution was added to each well. The plates were incubated for a further 45 minutes at 37°C. After incubation, 200 $\mu$l of CTAB solution was added to each well. Then, the absorbance at 600 nm was measured. An appropriate formula was used to convert the resulting data:

$$\%INHIBITION = \frac{T_S - T_C}{T_H - T_C} * 100\%$$

TS- hyaluronic acid + enzyme + extract
TC - hyaluronic acid + enzyme
TH - hyaluronic acid + extract

## average food intake

Diagram 3. Average food intake by bees [μL/bee]. Control — bees fed with 1:1 (w/v) aqueous sugar syrup. UAE.L - a sugar syrup with the addition of 0.1% extract obtained by ultrasound-assisted extraction. M.L — sugar syrup with the addition of 0.1% extract obtained by maceration.

Table 3. Survival of bees in study groups at the end of the experiment. At the beginning of the experiment, each group consisted of 200 individuals. Control - bees fed with 1:1 (w/v) aqueous sugar syrup. UAE.L0.01% - sugar syrup with the addition of 0.01% extract obtained by ultrasound-assisted extraction. UAE.L0.1% - sugar syrup with the addition of 0.1% extract obtained by ultrasound-assisted extraction. UAE.L1% - sugar syrup with the addition of 1% extract obtained by ultrasound-assisted extraction. UAE.L10% - sugar syrup with the addition of 10% extract obtained by ultrasound-assisted extraction. M.L0.01% - sugar syrup with the addition of 0.01% extract obtained by maceration. M.L0.1% - sugar syrup with the addition of 0.1% extract obtained by maceration. M.L1% - sugar syrup with the addition of 1% extract obtained by maceration. M.L10% - sugar syrup with the addition of 10% extract obtained by maceration.

| Bee survival in comparison to the control - the extract has no toxic effect. | |
|---|---|
| | Number of live bees at the end of experiment |
| control | 46 |
| UAE.L0.01 % | 48 |
| UAE.L0.1% | 49 |
| UAE.L1 | 45 |
| UAE.L10 | 43 |
| M.L0.01% | 51 |
| M.L0.1% | 48 |
| M.L1 | 46 |
| M.L10 | 42 |

Diagram 4. Level of bee nosema infection in the study groups at the end of the experiment. Control — bees fed with 1:1 (w/v) aqueous sugar syrup. UAE.L0.01% — sugar syrup with the addition of 0.01% extract obtained by ultrasound-assisted extraction. UAE.L0.1% — sugar syrup with the addition of 0.1% extract obtained by ultrasound-assisted extraction. UAE.L1% — sugar syrup with the addition of 1% extract obtained by ultrasound-assisted extraction. UAE.L10% — sugar syrup with the addition of 10% extract obtained by ultrasound-assisted extraction. M.L0.01% — sugar syrup with the addition of 0.01% extract obtained by maceration. M.L0.1% — sugar syrup with the addition of 0.1% extract obtained by maceration. M.L1% — sugar syrup with the addition of 1% extract obtained by maceration. M.L10% — sugar syrup with the addition of 10% extract obtained by maceration.

The level of infection is lowest in the ML10% group and is 2.05 million spores/bee compared to 15.20 million spores/bee in the control, indicating a strong therapeutic effect against honeybee nosema. The infection level for the UAE.L10% group is 4.20 million spores/bee compared to 15.20 million spores/bee in the control, indicating a therapeutic effect against honeybee nosema.

Research Example 1. Testing the toxicity of formulations according to the invention on bee organisms.

The preparations were administered to healthy bees that were placed in 45 standard cages - each contained 40 individuals, and observed over 20 days.

The study groups are:

1. Control - bees fed with 1:1 (w/v) aqueous sugar syrup.
2. UAE.L0.01% - sugar syrup with the addition of 0.01% extract obtained by ultrasound-assisted extraction.
3. UAE.L0.1% - sugar syrup with the addition of 0.1% extract obtained by ultrasound-assisted extraction.
4. UAE.L1% - sugar syrup with the addition of 1% extract obtained by ultrasound-assisted extraction.

5. UAE.L10% - sugar syrup with the addition of 10% extract obtained by ultrasound-assisted extraction.

6. M.L0.01% - sugar syrup with the addition of 0.01% extract obtained by maceration.

7. M.L0.1% - sugar syrup with the addition of 0.1% extract obtained by maceration.

8. M.L1% - sugar syrup with the addition of 1% extract obtained by maceration.

9. M.L10% - sugar syrup with the addition of 10% extract obtained by maceration.

After 20 days, the number of live bees in each cage was compared with the control.

The number of live bees in the research groups fed with the formulation according to the invention was comparable to the number of live bees in the control group, as shown in Table 3. In view of the natural mortality of bees, it must be concluded that the plant formulation according to the invention is a non-toxic substance and completely safe as an additive to bee food.

Study Example 2. Effect of the preparation according to the invention against nosema pathogens.

Efficacy of the preparation as an agent against pathogenic nosema pathogens was demonstrated by infecting bees with *Nosema (Vairimorpha)* spp. microsporidia and then, after the development of infection, by administering the preparation in different concentrations to several study groups. The bees were placed in 45 standard cages - 40 individuals each and observed over 20 days.

The bees were fed to form the following study groups:

1. Control - 5 cages with infected bees, fed throughout the experiment only with 1:1 (w/v) water sugar syrup.

2. UAE.L0.01% - 5 cages with infected bees, fed throughout the experiment with sugar syrup with the addition of 0.01% extract obtained by ultrasound-assisted extraction.

3. UAE.L0.1% - 5 cages with infected bees, fed throughout the experiment with sugar syrup with the addition of 0.1% extract obtained by ultrasound-assisted extraction.

4. UAE.L1% - 5 cages with infected bees, fed throughout the experiment with sugar syrup with the addition of 1% extract obtained by ultrasound-assisted extraction.

5. UAE.L10% - 5 cages with infected bees, fed throughout the experiment with sugar syrup with the addition of 10% extract obtained by ultrasound-assisted extraction.

6. M.L0.01% - 5 cages with infected bees, fed throughout the experiment with sugar syrup with the addition of 0.01% extract obtained by maceration.

7. M.L0.1% - 5 cages with infected bees, fed throughout the experiment with sugar syrup with the addition of 0.1% extract obtained by maceration.

8. M.L1% - 5 cages with infected bees, fed throughout the experiment with sugar syrup with the addition of 1% extract obtained by maceration.

9. M.L10% - 5 cages with infected bees, fed throughout the experiment with sugar syrup with the addition of 10% extract obtained by maceration.

The results of the experiments, shown in the figure as a Diagram 4, indicate a significantly reduced number of *Nosema (Vairimorpha)* spp. spores remaining in the bodies of infected bees administered after infection with the formulation according to the invention.

The level of infection is lowest in the ML10% group and amounts to 2.05 million spores/bee compared to 15.20 million spores/bee in the control, indicating a strong therapeutic effect against honeybee nosema. The infection level for the UAE.L10% group is 4.20 million spores/bee compared to 15.20 million spores/bee in the control, indicating a therapeutic effect against honeybee nosema.

Example II

Example II differs from Example I by the following data.

UAE extraction

Ethanol extract from a fresh lyophilized root of *Scutellaria baicalensis* obtained by UAE extraction technique to increase bee immunity and to combat nosema contains:

- TPC 51.42 mg/g of extract
- TFC 5.92 mg/g of extract
- TPAC 1.09 mg/g of extract,

where TPC stands for total polyphenols, TFC stands for total flavonoids and TPAC stands for total phenolic acids.

The method of obtaining an ethanol extract from fresh lyophilized root of *Scutellaria baicalensis* obtained by UAE extraction technique for increasing bee immunity and combating nosema consists of: extracting the ground root of *Scutellaria baicalensis* with 75% ethanol at 18°C for 16h, then using ultrasounds at 30 kHz for 10 min at 30°C, filtrating under reduced pressure to 0,1 bar and extracting with 75% ethanol by ultrasonication at 30 kHz for 10 min at 30°C, and re-extracting the extract obtained in this way three times in total, then vacuum-condensingin an evaporator at 20°C and lyophilizing the extract concentrated in such a way for 24h at -30°C, using 50 ml of ethanol for every 10 g of ground root.

Maceration.

Ethanol extract from fresh lyophilized root of *Scutellaria baicalensis* obtained by maceration technique to increase bee immunity and to combat nosema contains:

- TPC 58.78 mg/g of extract
- TFC 6.68 mg/g of extract
- TPAC 1.17 mg/g of extract

where TPC stands for total polyphenols, TFC stands for total flavonoids and TPAC stands for total phenolic acids.

The method of obtaining ethanol extract from fresh lyophilized root of *Scutellaria baicalensis* obtained by maceration technique for increasing bee immunity and combating nosema consists of extracting the ground root of *Scutellaria baicalensis* with 75% ethanol at 18°C for 16h, filtering under reduced pressure of 0.1 bar and extracting with 75% ethanol and then vacuum-condensing in an evaporator at 20°C, and lyophilising the extract concentrated in this way for 24h at -30°C, using 50 ml of ethanol for every 10 g of ground root.

Example III

Example III differs from Example I by the following data.
Ethanol extract from fresh lyophilized *Scutellaria baicalensis* root obtained by UAE extraction technique to increase bee immunity and combat nosema contains:

- TPC 51.42 mg/g of extract
- TFC 5.92 mg/g of extract
- TPAC 1.09 mg/g of extract,

where TPC stands for total polyphenols, TFC stands for total flavonoids and TPAC stands for total phenolic acids

- baicalein 0.155 mg/ml
- baicalin 0.244 mg/ml
- vogoside 0.234 mg/ml
- vogonoside 1.523 mg/ml

Method of obtaining an ethanol extract from fresh lyophilized root of *Scutellaria baicalensis* obtained by UAE extraction technique to increase bee immunity and to combat nosema consists of extracting the ground *Scutellaria baicalensis* root in 75% ethanol at 24°C for 36 hours, then subjecting to 50 kHz ultrasound for 25 minutes at 45°C, filtering under reduced pressure of 2 bar and extracting with 75% ethanol, subjecting to ultrasounds in the range of 50 kHz for 25 minutes at a temperature of 45°C, and repeating the whole procedure for the extract obtained in this way two to ten times, preferably three, then concentrating under vacuum in an evaporator at 45°C and freezing-drying the extract concentrated in this way over a period of 240 hours at -5°C, using 150 ml of ethanol for every 10 g of ground root.
Ethanol extract of fresh lyophilized root of *Scutellaria baicalensis* obtained by maceration technique to increase bee immunity and to combat nosema contains:

- TPC 58.78 mg/g of extract
- TFC 6.68 mg/g of extract

- TPAC 1.17 mg/g of extract

where TPC stands for total polyphenols, TFC stands for total flavonoids and TPAC stands for total phenolic acids

- baicalein 0.244 mg/ml
- baicalin 0.3423 mg/ml
- vogoside 0.487 mg/ml
- vogonoside 1.756 mg/ml.

The method of obtaining an ethanol extract from fresh lyophilized root of *Scutellaria baicalensis* obtained by maceration technique for increasing bee immunity and combating nosema consists of extracting the ground root of *Scutellaria baicalensis* with 75% ethanol at 24°C for 36h, filtering under reduced pressure of 2 bar and extracting with 75% ethanol, then vacuum-condensing in an evaporator at 45°C and lyophilizing the extract concentrated in this way for 240h at -5°C, using 50 ml of ethanol for every 10 g of ground root.

Example IV

Example IV differs from Example I by the following data.

Ethanol extract from fresh lyophilized *Scutellaria baicalensis* root obtained by UAE extraction technique to increase bee immunity and to combat nosema contains:

- TPC 51.42 mg/g of extract
- TFC 5.92 mg/g of extract
- TPAC 1.09 mg/g of extract,
- baicalein 0.290 mg/of ml
- baicalin 0.589 mg/ml
- vogoside 0.761 mg/ml
- vogonoside 2.601 mg/ml

Ethanol extract of fresh lyophilized root of *Scutellaria baicalensis* obtained by maceration technique to increase bee immunity and to combat nosema contains:

- TPC 58.78 mg/g of extract
- TFC 6.68 mg/g of extract
- TPAC 1.17 mg/g of extract

where TPC stands for total polyphenols, TFC stands for total flavonoids and TPAC stands for total phenolic acids

- baicalein 0.570 mg/ml
- baicalin 0.671 mg/ml
- vogoside 0.946 mg/ml
- vogonoside 2.683 mg/ml.

Example V.

Example V differs from Example I by the following data.

Ethanol extract from fresh lyophilized *Scutellaria baicalensis* root obtained by UAE extraction technique to increase bee immunity and to combat nosema contains:

- TPC 51.42 mg/g of extract
- TFC 5.92 mg/g of extract
- TPAC 1.09 mg/g of extract,

where TPC stands for total polyphenols, TFC stands for total flavonoids and TPAC stands for total phenolic acids

- baicalein 0.2 mg/ml
- baicalin 0.4 mg/ml
- vogoside 0.55 mg/ml
- vogonoside 2 mg/ml

Ethanol extract of fresh lyophilized root of *Scutellaria baicalensis* obtained by maceration technique to increase bee immunity and to combat nosema contains:

- TPC 58.78 mg/g of extract
- TFC 6.68 mg/g of extract
- TPAC 1.17 mg/g of extract

where TPC stands for total polyphenols, TFC stands for total flavonoids and
TPAC stands for total phenolic acids

- baicalein 0.4 mg/ml
- baicalin 0.4 mg/ml
- vogoside 0.61 mg/ml
- vogonoside 2.1 mg/ml.

## Claims

1. Ethanol extract from fresh lyophilized root of *Scutellaria baicalensis* for use in preparations increasing the immunity of bees and combating nosemosis.

2. Ethanol extract according to claim 1, **characterized in that** it is made from the roots of the *Scutellaria baicallensis* plant.

3. Ethanol extract from fresh lyophilized root of *Scutellaria baicallensis* obtained by the UAE extraction technique increasing the immunity of bees and combating nosemosis **characterized in that** it contains:

   - TPC 51.42 mg/g of extract
   - TFC 5.92 mg/g of extract
   - TPAC 1.09 mg/g of extract,

   where TPC stands for total polyphenols, TFC stands for total flavonoids and TPAC stands for total phenolic acids.

4. Alcoholic extract according to claim 3 **characterized in that** it contains:

   - baicalein from 0.155 to 0.290 mg/ml
   - baicalin from 0.244 to 0.589 mg/ml
   - vogoside from 0.234 to 0.761 mg/ml
   - vogonoside from 1.523 to 2.601 mg/ml

5. Method of obtaining an ethanol extract from a fresh lyophilized root of *Scutellaria baicalensis* obtained by UAE extraction technique to increase bees immunity and to combat nosemosis, **characterized in that** the ground *Scutellaria baicalensis* root is extracted with 75% ethanol at 18 to 24°C for 16 to 36 hours, preferably 24 hours, then subjected to ultrasounds in the range of 30 to 50 kHz, preferably 40 kHz, for 10 to 25 minutes, preferably 15 minutes, at a temperature of 30 to 45°C, preferably 37°C, filtered under reduced pressure in the range of 0.1 to 2 bar and extracted with 75% ethanol under ultrasonic treatment in the range of 30 to 50 kHz, preferably 40 kHz for 10 to 25 min, preferably 15 min at a temperature of 30 to 45°C, preferably 37°C, and the resulting extract is re-extracted two to ten times, preferably three, and then vacuum-concentrated in an evaporator at a temperature of 20 to 45°C, preferably 30°C, and the extract concentrated in this way is lyophilized for 24 to 240h, preferably 72h at a temperature of -30 to -5°C, preferably -15°C.

6. The method according to claim 5, **characterized in that** 50 to 150 ml, preferably 100 ml ethanol is used for every 10 g of ground root.

7. Ethanol extract from fresh lyophilized root of *Scutellaria baicalensis* obtained by maceration technique to increase bees immunity and to combat nosemosis, **characterized in that** it contains:

    - TPC 58.78 mg/g of extract
    - TFC 6.68 mg/g of extract
    - TPAC 1.17 mg/g of extract

    where TPC stands for total polyphenols, TFC stands for total flavonoids and TPAC stands for total phenolic acids.

8. Extract according to claim 7 **characterized in that** it contains:

    - baicalein from 0.244 to 0.570 mg/ml
    - baicalin from 0.3423 to 0.671 mg/ml
    - vogoside from 0.487 to 0.946 mg/ml
    - vogonoside from 1.756 to 2.683 mg/ml.

9. Method of obtaining an ethanol extract from fresh lyophilized root of *Scutellaria baicalensis* by maceration technique to increase bees immunity and to combat nosemosis, **characterized in that** the ground root of *Scutellaria baicalensis* is extracted with 75% ethanol at 18 to 24°C for 16 to 36h, preferably 24h, is filtered under reduced pressure of 0.1 to 2 bar and extracted with 75% ethanol and then vacuum-condensed in an evaporator at 20 to 45°C, preferably 30°C, and the concentrated extract is lyophilized for 24 to 240h, preferably 72h at -30 to -5°C, preferably -15°C.

10. Method according to claim 9 **characterized in that** 50 to 150 ml, preferably 100 ml of ethanol is used for every 10 g of ground root.

Europäisches
Patentamt

European
Patent Office

Office européen
des brevets

# EUROPEAN SEARCH REPORT

**Application Number**

EP 25 15 4974

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LU YINGJIAN ET AL: "Study of the Chemical Composition and Antimicrobial Activities of Ethanolic Extracts from Roots of Scutellaria baicalensis Georgi", JOURNAL OF AGRICULTURAL AND FOOD CHEMISTRY, vol. 59, no. 20, 1 October 2011 (2011-10-01), pages 10934-10942, XP093294083, US ISSN: 0021-8561, DOI: 10.1021/jf202741x | 3-10 | INV. A01N65/22 A01P3/00 |
| A | * abstract * * Preparation of SBG ethanol extracts; page 10935, left-hand column * * page 10938; table 1 * * page 10940 * | 1,2 | |
| Y | ZWOLAN ADAM ET AL: "Characteristics of water and ethanolic extracts of Scutellaria baicalensis root and their effect on color, lipid oxidation, and microbiological quality of chicken meatballs during refrigerated storage", JOURNAL OF FOOD PROCESSING AND PRESERVATION, vol. 46, no. 1, 1 January 2022 (2022-01-01), XP093294092, TRUMBULL, CT, US ISSN: 0145-8892, DOI: 10.1111/jfpp.16192 | 3-10 | |
| | | | TECHNICAL FIELDS SEARCHED (IPC) |
| | | | A01N A01P |
| A | * abstract * * page 2, left-hand column, line 33 - right-hand column, line 5 * | 1,2 | |

-/--

The present search report has been drawn up for all claims

| Place of search | Date of completion of the search | Examiner |
|---|---|---|
| Munich | 22 July 2025 | Hateley, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

**EUROPEAN SEARCH REPORT**

Application Number

EP 25 15 4974

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | YOU J ET AL: "On-line continuous flow ultrasonic extraction coupled with high performance liquid chromatographic separation for determination of the flavonoids from root of Scutellaria baicalensis Georgi", JOURNAL OF CHROMATOGRAPHY A, ELSEVIER, AMSTERDAM, NL, vol. 1217, no. 12, 19 March 2010 (2010-03-19), pages 1875-1881, XP026921320, ISSN: 0021-9673, DOI: 10.1016/J.CHROMA.2010.01.050 [retrieved on 2010-01-25] | 3-10 | |
| A | * abstract * <br> * figure 1 * <br> * 4. Conclusion; page 1880 * | 1,2 | |
| Y | CN 101 961 384 A (UNIV ZHEJIANG; CHIATAI QINGCHUNBAO PHARMACEUTICAL CO LTD) 2 February 2011 (2011-02-02) * page 1 - page 3 * | 3-10 | TECHNICAL FIELDS SEARCHED (IPC) |
| A | DA XIA ET AL: "Antifungal activity and mechanism of action of Ou-gon (Scutellaria root extract) components against pathogenic fungi", SCIENTIFIC REPORTS, vol. 9, no. 1, 1 February 2019 (2019-02-01), pages 1683-1683, XP093294102, US ISSN: 2045-2322, DOI: 10.1038/s41598-019-38916-w * the whole document * | 1-10 | |

-/--

The present search report has been drawn up for all claims

| Place of search | Date of completion of the search | Examiner |
|---|---|---|
| Munich | 22 July 2025 | Hateley, Martin |

EPO FORM 1503 03.82 (P04C01)

Europäisches
Patentamt

European
Patent Office

Office européen
des brevets

**EUROPEAN SEARCH REPORT**

Application Number

EP 25 15 4974

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JP H05 103605 A (KOJUNDO KAGAKU KENKYUSHO KK) 27 April 1993 (1993-04-27) * the whole document * | 1-10 | |
| A | DUMITRU ADRIAN ET AL: ""IN VITRO" STUDIES ON USING NATURAL ESSENTIAL OILS IN TREATMENT OF NOSEMOSIS IN HONEY BEES: DETERMINATION OF THE THERAPEUTIC DOSE", SCIENTIFIC WORKS. SERIES C. VETERINARY MEDICINE., vol. LXIII, no. 2, 1 January 2017 (2017-01-01), pages 165-170, XP093294226, ISSN: 2065-1295 Retrieved from the Internet: URL:2017> * abstract * * page 166, left-hand column, lines 25-44 * * page 168 - page 169; tables 1-2 * * Conclusions; page 170 * | 1-10 | |

TECHNICAL FIELDS
SEARCHED (IPC)

The present search report has been drawn up for all claims

| Place of search | Date of completion of the search | Examiner |
|---|---|---|
| Munich | 22 July 2025 | Hateley, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................................

& : member of the same patent family, corresponding document

EPO FORM 1503 03.82 (P04C01)

page 3 of 3

## ANNEX TO THE EUROPEAN SEARCH REPORT
## ON EUROPEAN PATENT APPLICATION NO.

EP 25 15 4974

This annex lists the patent family members relating to the patent documents cited in the above-mentioned European search report.
The members are as contained in the European Patent Office EDP file on
The European Patent Office is in no way liable for these particulars which are merely given for the purpose of information.

22-07-2025

| Patent document cited in search report | Publication date | Patent family member(s) | Publication date |
|---|---|---|---|
| CN 101961384 A | 02-02-2011 | NONE | |
| JP H05103605 A | 27-04-1993 | NONE | |

EPO FORM P0459

For more details about this annex : see Official Journal of the European Patent Office, No. 12/82

**REFERENCES CITED IN THE DESCRIPTION**

**Patent documents cited in the description**

- PL 241381
- PL 241380
- PL 231692
- PL 232685